# EUROPÄISCHE PATENTSCHRIFT

(11) **EP 0 879 033 B1**
(45) Veröffentlichungstag und Bekanntmachung des Hinweises auf die Patenterteilung: **18.06.2003**
(21) Anmeldenummer: 97900601.2
(22) Anmeldetag: 14.01.1997
(51) Int. Cl.: A61F 5/02

(54) **LORDOSEKORREKTURMIEDER**
LORDOSIS CORRECTION CORSET
CORSET DE CORRECTION DE LORDOSE

(30) Priorität: 09.02.1996 DE 19604740
(43) Veröffentlichungstag der Anmeldung: 25.11.1998
(73) Patentinhaber: Lüssenhop, Stefan, Dr., 37444 St. Andreasberg (DE); Soyka, Matthias, Dr., 21031 Hamburg (DE)
(72) Erfinder: LÜSSENHOP, Stefan, 37444 St. Andreasberg (DE); SOYKA, Matthias, 21031 Hamburg (DE); NELSON, Ronald, E., Chetek, WI 54728 (US)
(74) Vertreter: Schaefer, Konrad, Dipl.-Phys.
(86) Internationale Anmeldenummer: EP9700133
(87) Internationale Veröffentlichungsnummer: WO97028763

(56) Entgegenhaltungen:
- DE-A- 4 415 604
- FR-A- 2 106 247
- US-A- 4 691 696
- US-A- 5 205 815
- US-A- 5 470 000

## Beschreibung

Die Erfindung betrifft ein Lordosekorrekturmieder.

Von der Seite gesehen, ist die menschliche Wirbelsäule 5-förmig gekrümmt mit einer Auswölbung nach hinten (Kyphose) im Bereich der Brustwirbelsäule und mit einer Vorwölbung nach vorn (Lordose) im Bereich der Lendenwirbelsäule. In letzterem Bereich ist die Beweglichkeit am größten, und es kommt dort am häufigsten zu Rückenbeschwerden, z.B. aufgrund von Bandscheibenvorfällen. Jede Fehlstellung, insbesondere eine zu starke Lordose, ist dann schmerzhaft. Die korrekte schmerzfreie Stellung kann durch Training der die Wirbelsäule haltenden Muskulatur erreicht werden. Da Gymnastik unbeliebt ist, werden Mieder verwendet, die sich zu diesen Zwecken großer Verbreitung erfreuen.

Den Bauch umschließende Mieder erhöhen den Bauchinnendruck, so daß der Bauch von innen gegen die Wirbelsäule drückt und diese aus zu starker Lordose in die Normalstellung bringt. Eine Pelotte (Formschale), hinter der Wirbelsäule getragen, kann die Einhaltung der gewünschten Wirbelsäulenstellung gewährleisten.

Lordosekorrekturmieder sind aus
DE 21 43 781 C3 (Fig. 6),
FR-PS 617 964,
DE 21 43 787 C3,
DE 88 09 297 U1 (Fig. 3),
und aus Firmenprospekt
"MIKROS"
Vorbeugen und Heilen mit System
Ing. Harald Warncke GmbH,
Saseler Bogen 2, D-22393 Hamburg,
bekannt.

Die beiden erstgenannten Schriften zeigen orthopädische Mieder zur Korrektur der Wirbelsäulenstellung, der letztgenannte Prospekt einen vorbeugend zur Vermeidung von Wirbelsäulenerkrankungen bei körperlich hochbelasteten Arbeitern verwendetes Mieder.

Gemeinsam ist diesen bekannten Konstruktionen eine im Höhenbereich der Lendenwirbelsäule den Leib umschließende Binde mit auf dieser angeordneten Gurten, die seitlich den Bauch umschlingen und auf dem Bauch unter Vorspannung individuell befestigbar sind. Dadurch kann die Binde lose und bequem gehalten sein. Die Bauchpressung kann über individuell einstellbare Gurtspannung von Fall zu Fall reguliert werden. Es kann also beispielsweise im Sitzen durch Öffnen der Gurte der Bauch entlastet werden und im Stand oder bei schweren Arbeiten durch Befestigen der Gurte unter hoher Spannung ein hoher Bauchdruck eingestellt werden.

Bei den bekannten Konstruktionen verlaufen die Gurte entweder waagerecht oder im vorderen Bereich auf dem Bauch leicht abwärts verlaufend nur jeweils auf einer Körperhälfte. Sie sind jeweils auf dem Bauch mit ihren vorderen Enden vor der Körpermitte befestigt oder in der Körpermitte miteinander verbunden. Ihre Wirkung besteht lediglich darin, die Bauchpressung zu erhöhen.

Aus der
FR-PS 536 016
ist bei einem Mieder auch eine Variante mit den Leib voll umschlingendem Gurt bekannt.

In der
DE 17 66 250 A1
sind die Nachteile solcher Korsetts dargelegt. Da durch die Bauchpressung die Lordose passiv aufgerichtet wird, wird die zur Aufrichlung der Lordose dienende Muskulatur von ihrer Arbeit entlastet. Da bei längerem Tragen des Korsetts die Muskulatur ständig unbelastet bleibt, verkümmert sie, so daß langlristig durch Schädigung der die Wirbelsäule stützenden Muskulatur die Haltung der Wirbelsäule verschlechtert wird. Der Patient kann schließlich ohne Korsett nicht mehr leben.

In der letztgenannten Schrift wird ein ganz anderer Weg vorgeschlagen, die Wirbelsäule zu korrigieren, nämlich durch dauernde Aktivierung der Rückenmuskulatur mittels eines Gürtels, der durch Druckreiz auf die Muskeln diese in aus der medizinischen Literatur bekannter Weise anregt, um dadurch die Wirbelsäulenstellung zu verbessern.

Mit dieser bekannten Konstruktion kann über die Aktivierung der Rückenmuskeln zum Ausgleich der Lordose aber nur wenig beigetragen werden, da die Lordose nur durch Erhöhung des Bauchdruckes ausgleichbar ist. Hierfür zuständig sind die Bauchmuskeln und insbesondere die schrägen Bauchmuskeln, die für die Ausbildung der Taille und somit für den Bauchinnendruck verantwortlich sind. Hierzu wird verwiesen auf
Benninghoff/Goertler
"Lehrbuch der Anatomie des Menschen".
1. Band von Jochen Staubesand,
Urban & Schwarzenberg, München, Wien,
Baltimore 1978
Seiten 280 - 289, insbesondere Seiten 285 und 286
sowie
"Funktionelle Anatomie der Gelenke"
von I. A. Kapandji,
Band 3,
Ferdinand Enke Verlag Stuttgart 1992,
Seiten 80 - 95, Abb. auf Seite 93.

Die bekannten Mieder wirken sämtlich auf die Bauchmuskulatur erschlaffend, weil sie sie ohne Anregung entlasten und den Bauchdruck nur passiv erhöhen.

Das
DE-GM 1 700 630
zeigt ein Mieder mit auf der Binde angeordneten längselastischen Gurten, die über den Bauch gekreuzt schräg abwärts verlaufen. Das obere Ende eines jeden Gurtes ist auf der einen Seitennaht und das untere Ende auf der anderen Seitennaht befestigt. Unter dem einen der Gurte ist in einer Tasche der Binde eine Hemienpelotte angeordnet, die von diesem Gurt und zusätzlich mit einem Schenkelgurt angepreßt wird. Diese Konstruktion dient zum Rückhalten eines Leistenbruches. Die Gurte dienen hier der Aufrechterhaltung des Preßdruckes auf den Leistenbruch.

Aus der
FR-A-2106247
ist ein auch für Lordosekorrekturzwecke verwendbares Mieder der im Oberbegriff des Anspruch 1 genannten Art bekannt, bei dem die vorderen Enden der beiden Gurte auf dem Bauchteil der Binde im mittleren Bereich angreifen. Die Gurte sollen hier im seitlichen Bereich so verlegt sein, daß sie über den Nieren verlaufen. Bei geeigneter Materialwahl soll durch Materialreibung und daraus folgende elektrostatische Aufladung ein auf die Nieren wirkender Effekt erzielt werden.

Diese Konstruktion hat möglicherweise auch eine die Lordose korrigierende Wirkung, jedoch wie beim vorgenannten Stand der Technik mit dem Nachteil, daß eine rein passive Bauchstützung erfolgt, die bei längerem Tragen zur Muskelerschlaffung führt.

Die Aufgabe der vorliegenden Erfindung besteht darin, ein Lordosekorrekturmieder zu schaffen, das durch Aktivierung insbesondere der schrägen Bauchmuskulatur langfristig zur Verbesserung des Zustandes führt.

Diese Aulgabe wird erfindungsgemäß mit den Merkmalen des Anspruches 1 gelöst.

Bei dem erfindungsgemäßen Mieder verlaufen die vorderen Endbereiche der Gurte im wesentlichen in Richtung der schrägen Bauchmuskulatur, die jeweils von den Rippen der einen Körperseite zum Beckenkamm der anderen Körperseite verläuft. Die Gurte greifen mit ihren vorderen Enden, dem Verlauf dieser Muskulatur folgend, im Bereich der unteren Muskeln an und ergeben durch ihre Zugwirkung auf der Körperoberfläche oberhalb der Muskeln auf diese einen aktivierenden Reiz. Die Erfindung macht sich die Erkenntnis zunutze, daß der in
"Kinder-Orthopädie"
von Rupprechl Bernbeck und Günter Dahmen,
3. Auflage 1983
Georg Thieme Verlag Stuttgart - New York,
Seite 232, Abb. 160
beschriebene Bauchhebegriff korrekt so auszuführen ist, daß die Hände bei überkreuzten Unterarmen jeweils auf der gegenüberliegenden Leistengegend, also im Bereich der unteren Ansätze der schrägen Bauchmuskeln liegen. Mit dem erfindungsgemäßen Mieder wird einerseits in bekannter Weise passiv der Bauchdruck erhöht, zusätzlich aber die schräge Bauchmuskulatur angeregt und somit eine wirksame Dauerverbesserung der Bauchspannung und darüber der Rückbildung der Lordose erreicht.

Vorteilhaft sind die Merkmale des Anspruches 2 vorgesehen. Durch den hohen hinteren Ansatz der Gurte wird ihre Schrägführung im wesentlichen in Richtung der unteren Anteile der schrägen Bauchmuskulatur ermöglicht, und es erfolgt der hintere Angriff im oberen Bereich der Lordose, so daß diese nicht durch Druck von hinten verstärkt wird.

Vorteilhaft sind die Merkmale des Anspruches 3 vorgesehen. In bekannter Weise kann eine solche Verstärkung, gegebenenfalls noch unterstützt durch eine Pelotte (Hartschale), das zu starke Ausweichen der Wirbelsäule nach hinten verhindern und somit die Einstellung der gewünschten Lage gewährleisten.

Vorteilhaft sind die Merkmale des Anspruches 4 vorgesehen. Beim Anlegen der Gurte sorgen die Korrekturbänder dafür, daß die Gurte im seitlichen Bereich hochgehalten bleiben, so daß im vorderen Bereich über dem Bauch die gewünschte Schräglage in Richtung der schrägen Bauchmuskulatur eingestellt wird.

Vorteilhaft sind die Merkmale des Anspruches 5 vorgesehen. Auf diese bewährte Weise lassen sich die Gurte in individuell wählbarer Spannung anlegen und leicht wieder lösen.

In den Zeichnungen ist die Erfindung beispielsweise und schematisch dargestellt. Es zeigen:
- Fig. 1: ein erfindungsgemäßes Mieder, an einem Körper getragen in Frontalansicht,
- Fig. 2: das Mieder der Fig. 1 in Seitenansicht und
- Fig. 3: in schematischer Frontalansicht eines Skeletts die schräge Bauchmuskulatur und die Gurte des erfindungsgemäßen Mieder.

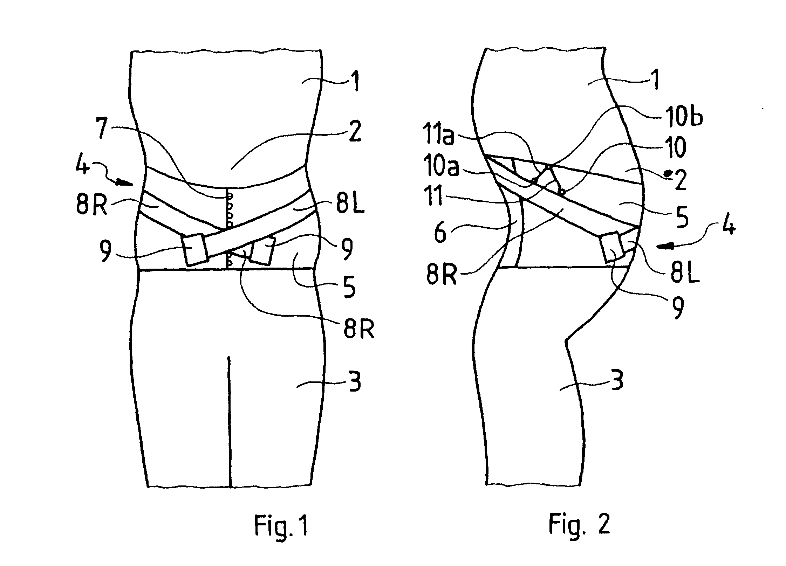

In den Fig. 1 und 2 ist in Frontal- und Seitenansicht ein stehender menschlicher Körper dargestellt mit Brust 1, Bauch 2 und Beinen 3. Wie Fig. 2 zeigt, besteht eine starke Lordose, also Vorwölbung, im Bereich der Lendenwirbelsäule. Diese soll mit dem dargestellten Mieder 4 ausgeglichen werden.

Das Mieder 4 wird um den Bauch 2 angelegt und besteht aus einer schlauchförmig den Bauch umgebenden Binde 5 aus üblicherweise für solche Zwecke verwendetem stretchbarem Material. Am Rücken kann eine Verstärkung 6 vorgesehen sein, die an die Binde angearbeitet oder in diese eingearbeitet sein kann und der Wirbelsäule im Bereich der Lendenwirbelsäule die gewünschte Krümmung (Lordose) vorgibt. Die Verstärkung 6 kann starr ausgebildet sein, beispielsweise als Harlschale, oder auch federnd oder biegbar. Sie kann im einfachsten Fall aus in die Binde 5 eingearbeiteten, parallel zur Wirbelsäule verlaufenden Stäben (Korsettstäben) bestehen. Zusätzlich kann eine breitere Pelolle, z.B. abnehmbar, vorgesehen sein, die die Wirkung der Verstärkung 6 unterstützt. Die Binde 5 ist vorn mit einem Verschluß 7, z.B. einem Reißverscliluß oder Haftverschluß, zu öffnen.

Die Binde 5 kann relativ locker und bequem um den Bauch getragen werden. Zu diesem Zweck kann, auch zur Anpassung an unterschiediche Bauchweiten, der Verschluß 7 umfangsverslellbar ausgebildet sein.

Es sind zwei Gurle 8L und 8R vorgesehen, die im oberen Bereich der Binde 5 mittig auf dem Rücken befestigt sind und von dort schräg abwärts nach vorn, auf dem Bauch gekreuzt bis zur gegenüberliegenden Bauchseite verlaufen. An ihren vorderen Enden sind Haftverschlüsse 9 vorgesehen, die auf der geeignet haftfähig ausgebildeten Oberfläche der Binde 5 bzw. des anderen Gurtes 8R haftend befestigt werden können. Die Gurte 8L und 8R sind längselastisch spannbar und können in individuell gewünschte Spannung gebracht und mit den Haftverschlüssen 9 befestigt werden.

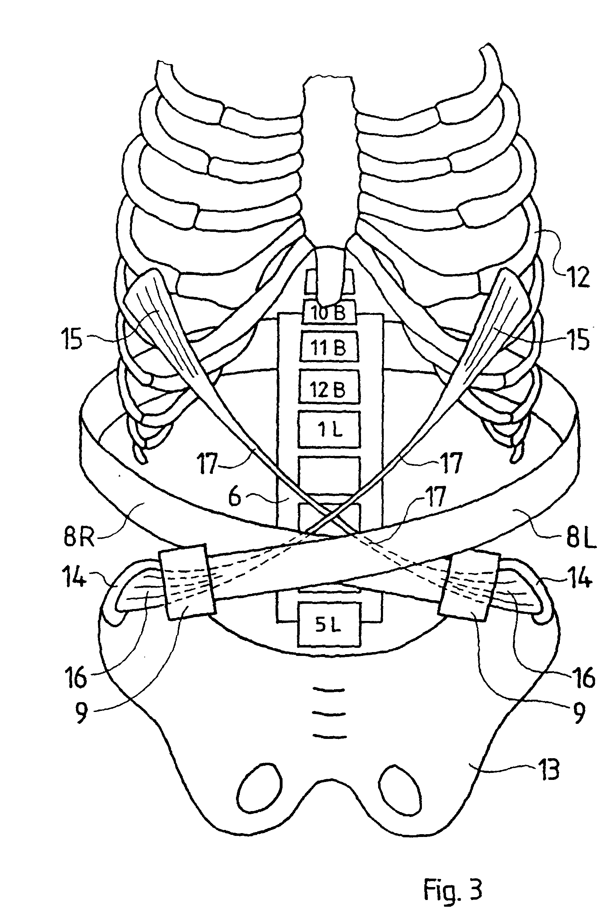

Wie Fig. 2 zeigt, ist jeder der Gurte 8L und 8R seitlich an seinem oberen Rand bei 10 und 10a mit einem Korreklurband 11, 11a verbunden, die bei 10b am oberen Rand der Binde 5 in einem gemeinsamen Punkt befestigt sind. Die Korrekturbänder 11, 11a sorgen dafür, daß die Gurle 8L und 8R seitlich nicht zu tief sitzen, also vorn über dem Bauch die in Fig. 1 dargestellte schräge Lage einnehmen. Unter Umständen reicht auch jeweils eines der beiden dargestellten Korrekturbänder 11, 11a, vorzugsweise das Korrekturband 11, aus. Die gewünschte schräge Lage, die in den Figuren dargestellt ist, kann ferner dadurch gesichert werden, daß die Oberfläche der Binde 5 und des Gurtes 8R nur in dem Bereich haftlähig, also zur Aufnahme der Haftverschlüsse 9 geeignet ausgebildet ist, die der in Fig. 1 dargestellten korrekten Lage der vorderen Enden der Gurte 8L und 8R entspricht. Es ergibt sich dadurch eine Lage der Gurte 8L und 8R, wie sie in Fig. 3 dargestellt ist.

Fig. 3 zeigt ausschnittsweise das Skelett des in Fig. 1 dargestellten Körpers in derselben Darstellung, also in Frontalansicht. Man erkennt den Brustkorb mit der Rippenanordnung 12 und das Becken 13 mit dem Beckenkamm 14. In starker Schematisierung ist die wesentliche Komponente der schrägen Bauchmuskulatur dargestellt, bestehend aus dem an den Rippen ansetzenden musculus obliquus externus 15 und dem musculus obliquus internus 16, der auf der gegenüberliegenden Seite am Beckenkamm 14 ansetzt. Die jeweils gegenüberliegend zusammenarbeitenden Muskelanteile 15 und 16 sind durch Sehnenzüge 17 verbunden. Es wird darauf hingewiesen, daß die außerordentlich komplexen anatomischen Verhältnisse im Bereich der schrägen Bauchmuskulatur in Fig. 3 extrem stark schematisiert dargestellt sind. Die beiden dargestellten Muskelanteile 15, 17, 16 stellen jeweils nur den mittleren charakteristischen Anteil einer Muskelschlinge der schrägen Bauchmuskulatur dar, die jeweils noch weitere parallele solcher Anteile aufweist.

Fig. 3 zeigt ferner in starker Schematisierung die Wirbelsäule mit Angaben der Wirbelposition (zehnter Brustwirbel 10B, elfter Brustwirbel 11B, zwölfter Brustwirbel 12B, erster Lendenwirbel 1L, fünfter Lendenwirbel 5L). Die Lendenwirbelsäule liegt also zwischen 1L und 5L. Der Übergangsbereich zwischen der Brustwirbelsäule und der Lendenwirbelsäule liegt zwischen 12B und 1L.

Das in den Fig. 1 und 2 dargestellte Mieder 4 ist teilweise, unter Weglassung der Binde 5 dargestellt. Es sind nur die Verstärkung 6 und die Gurte 8L und 8R dargestellt.

Aus Fig. 3 ist zu erkennen, daß die Gurte 8R und 8L hinten auf dem Rücken im unteren Bereich der Brustwirbelsäule, also vorzugsweise im Bereich etwa des zehnten Brustwirbels 10B ansetzen, schräg abwärts gerichtet den Bauch 2 umlaufen und mit ihren vorderen Enden 9 jeweils im Beieich der Leistengegend, also über dem musculus obliquus internus 16 angreifen.

Durch den Zugangriff der Gurte an den mit den Kreisen 9 bezeichneten Stellen über dem jeweiligen unteren Muskelanleil 16 der schrägen Bauchinuskulatur wird diese im Ganzen stimuliert. Durch die gekreuzte Lage der schrägen Bauchmuskeln ergibt sich die aus der Literatur ersichtliche taillenlormige Einziehung des Bauches, die über Erhöhung der Bauchinnenspannung die Wirbelsäule nach hinten gegen die Verstärkung 6 anlegt und dadurch eine zu starke Lordose ausgleicht.

## Patentansprüche

1. Lordosekorrekturmieder (4) mit einer zum Umschließen des Bauches (2) eines Patienten ausgebildeten schlauchförmigen Binde (5) mit zwei längselastisch ausgebildeten Gurten (8L, 8R), die mit ihren hinteren Enden auf dem dem Rücken des Patienten anzulegenden Rückenteil der Binde (5) befestigt sind und von dort auf der Außenseite der Binde (5) schräg abwärts bis zu dem dem Bauch (2) des Patienten anzulegenden Bauchteil der Binde (5) verlaufen, wo sie mit ihren vorderen Enden (9) lösbar befestigt sind, **dadurch gekennzeichnet, daß** die Gurte (8L, 8R) mit ihren hinteren Enden auf dem oberen Bereich des Rückenteiles der Binde (5) befestigt sind und daß sich die Gurte (8L, 8R) über dem Bauchteil der Binde (5) kreuzen und mit ihren vorderen Enden (9) auf der Binde (5) in den Bereichen des Bauchteiles, die in der Leistengegend dem musculus obliquus internus des Patienten anzulegen sind, befestigt sind, wobei das vordere Ende (9) des einen Gurtes (8R) direkt auf der Binde (5) und das vordere Ende (9) des anderen Gurtes (8L) entweder direkt oder indirekt über den zwischenliegenden anderen Gurt (8R) auf der Binde (5) befestigt ist.

2. Mieder nach Anspruch 1, **dadurch gekennzeichnet, daß** die hinteren Enden der Gurte (8L, 8R) auf dem dem unteren Bereich (10B - 12B) der Brustwirbelsäule anzulegenden Bereich der Binde (5) befestigt sind.

3. Mieder nach Anspruch 1, **dadurch gekennzeichnet, daß** die Binde (5) mit einer die Lendenwirbelsäule (1L - 5L) stützenden Verstärkung (6) versehen ist.

4. Mieder nach Anspruch 1, **dadurch gekennzeichnet, daß** auf jeder Seite der Binde (5) wenigstens ein in angelegter Lage der Gurte (8L, 8R) gestrammtes Korrekturband (11, 11a) vorgesehen ist, das mit seinem oberen Ende (10b) im Bereich des oberen Randes der Binde (5) und mit seinem unteren Ende (10, 10a) im Bereich des oberen Randes des Gurtes (8L, 8R) befestigt ist.

5. Mieder nach Anspruch 1, **dadurch gekennzeichnet, daß** die vorderen Enden der Gurte mit Haftverschlüssen (9) auf der entsprechend haftfähig ausgerüsteten Oberfläche der Binde (5) oder des anderen Gurtes (8R) befestigbar sind.

## Claims

1. Corsage (4) for the correction of lordosis, comprising a tube-shaped bandage (5) designed for embracing the abdomen (2) of a patient, with two lengthwise resilient belts (8L, 8R) affixed with their distal ends to the posterior part of the bandage (5) that is to be attached to the back of the patient, and extending downwards in an inclined way on the outer side of the bandage (5) to the anterior part of the bandage (5) that is to be attached to the abdomen (2) of the patient where they are reversibly affixed with their proximal ends (9), **characterized in that** the belts (8L, 8R) are affixed with their distal ends to the upper area of the posterior part of the bandage (5), and that the belts (8L, 8R) intersect above the anterior part of the bandage (5) and are affixed with their proximal ends to the bandage (5) in these areas of its anterior part which are to be attached to the patient's musculus obliquus internus in the groin area, wherein the proximal end (9) of one belt (8R) is affixed directly to the bandage (5) and the proximal end (9) of the other belt (8L) is affixed to the bandage (5) either directly or indirectly via the interjacent other belt (8R).

2. Corsage according to claim 1, **characterized in that** the distal ends of the belts (8L, 8R) are affixed to the area of the bandage (5) that is to be attached to the lower area (10B - 12B) of the thoracic spine.

3. Corsage according to claim 1, **characterized in that** the bandage (5) is provided with a fortification supporting the lumbar spine (1L-5L).

4. Corsage according to claim 1, **characterized in that** on both sides of the bandage (5) at least a single correction belt (11, 11a) is provided being tight when the belts (8L, 8R) are in wearing position, which is affixed with its upper end (10b) in the area of the upper edge of the bandage (5), and which is affixed with its lower end (10, 10a) (8L, 8R) in the area of the upper edge of the belt.

5. Corsage according to claim 1, **characterized in that** the anterior ends of the belts can be affixed with adhesive breeches to the surface of the bandage (5) designed accordingly adhesive.

## Revendications

1. Corset de correction de lordose (4) muni d'un bandage (5) de forme tubulaire conformé de façon à entourer le ventre (2) d'un patient, et de deux sangles (8L, 8R) élastiques dans leur sens longitudinal, lesquelles sont fixées par leur extrémité arrière sur la partie dorsale du bandage (5) appliquée au dos du patient d'où elles passent, en biais vers le bas, sur la face extérieure du bandage (5) jusqu'à sa partie ventrale appliquée au ventre (2) du patient où elles sont fixées de façon amovible par leur extrémité avant (9), **caractérisé en ce que** les sangles (8L, 8R) sont fixées par leur extrémité arrière à la zone supérieure de la partie dorsale du bandage (5) et **en ce que** les sangles (8L, 8R) sont croisées sur la partie ventrale du bandage (5) pour être fixées par leur extrémité avant (9) aux zones de la partie ventrale à appliquer dans la région inguinale contre le muscle oblique interne du patient, l'extrémité avant (9) de l'une des sangles (8R) étant directement fixée sur le bandage (5) et l'extrémité avant (9) de l'autre sangle (8L) étant fixée soit directement, soit indirectement par-dessus la première sangle (8R) se trouvant entre la seconde et le bandage (5).

2. Corset selon la revendication 1, **caractérisé en ce que** les extrémités arrières des sangles (8L, 8R) sont fixées à la partie de la sangle (5) à appliquer contre la région inférieure (10B - 12B) de la colonne vertébrale thoracique.

3. Corset selon la revendication 1, **caractérisé en ce que** la sangle (5) est munie d'un renforcement (6) destiné à soutenir la colonne vertébrale lombaire (1L-5L).

4. Corset selon la revendication 1, **caractérisé en ce qu'**est prévue, de chaque côté du bandage (5), au moins une bande de correction (11, 11a) tendue lorsque les sangles (8L, 8R) sont en place, cette bande étant fixée par son extrémité supérieure (10b) au niveau du bord supérieur du bandage (5) et par son extrémité inférieure (10, 10a) au niveau du bord supérieur de la sangle (8L, 8R).

5. Corset selon la revendication 1, **caractérisé en ce que** les extrémités avant des sangles peuvent être fixés au moyen de fermetures adhérentes (9) sur la surface traitée de façon à permettre une telle adhésion du bandage (5) ou de l'autre sangle (8R).
